(19)

**Europäisches Patentamt**
**European Patent Office**
**Office européen des brevets**

(11) **EP 1 521 879 B1**

(12) **EUROPÄISCHE PATENTSCHRIFT**

(45) Veröffentlichungstag und Bekanntmachung des Hinweises auf die Patenterteilung:
**19.03.2008 Patentblatt 2008/12**

(21) Anmeldenummer: **03763853.3**

(22) Anmeldetag: **15.07.2003**

(51) Int Cl.:
***D04H 1/56*** *(2006.01)*

(86) Internationale Anmeldenummer:
**PCT/EP2003/007628**

(87) Internationale Veröffentlichungsnummer:
**WO 2004/007826 (22.01.2004 Gazette 2004/04)**

(54) **KOSMETIKPAD UND VERFAHREN ZU SEINER HERSTELLUNG**

COSMETIC PAD AND METHOD FOR THE PRODUCTION THEREOF

RONDELLE COSMETIQUE ET PROCEDE DE FABRICATION DE LADITE RONDELLE

(84) Benannte Vertragsstaaten:
**AT BE BG CH CY CZ DE DK EE ES FI FR GB GR HU IE IT LI LU MC NL PT RO SE SI SK TR**

(30) Priorität: **15.07.2002 EP 02400033**

(43) Veröffentlichungstag der Anmeldung:
**13.04.2005 Patentblatt 2005/15**

(73) Patentinhaber: **Paul Hartmann Aktiengesellschaft 89522 Heidenheim (DE)**

(72) Erfinder:
• **MANGOLD, Rainer**
**89542 Herbrechtingen (DE)**

• **RÖMPP, Angela**
**73119 Zell u.A. (DE)**
• **GERHARTL, Gerd**
**CH-9115 Dicken (CH)**

(74) Vertreter: **Friz, Oliver**
**Patentanwälte,**
**Dreiss, Fuhlendorf, Steimle & Becker,**
**Postfach 10 37 62**
**70032 Stuttgart (DE)**

(56) Entgegenhaltungen:
**EP-A- 1 136 024       EP-A- 1 283 019**
**FR-A- 2 276 030       US-A- 4 459 987**
**US-A- 6 017 351**

EP 1 521 879 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein zum Reinigen der Haut insbesondere zum Peeling geeignetes Kosmetikpad und ein Verfahren zur Herstellung dieses Kosmetikpads.

Zum Peeling geeignete Kosmetikpads, das heißt insbesondere Kosmetikpads, welche eine als weich empfundene Seite aufweisen, und eine raue Seite, die zum "Peeling", also zum Entfernen von abgestorbenen Hautschuppen verwendet wird, sind bereits bekannt geworden.

So offenbart EP 1136024 A2 ein Peeling-Pad, das eine durchgehende Kunststoffbeschichtung aufweist. Die Kunststoffbeschichtung kann aus einem in flüssigem Zustand auf das Kosmetikpad aufgebrachten und dort ausgehärteten Kunststoff bestehen. In den Kunststoff ist ein Schleifmittel eingearbeitet. Von Nachteil ist hier insbesondere die Gefahr des unerwünscht tiefen Eindringens des noch flüssigen Kunststoffes in den Faserverbund des Kosmetikpads, bevor der Kunststoff ausgehärtet ist. In einer anderen Ausführungsform ist die Kunststoffbeschichtung als fertige Folie mit abrasiver Oberfläche mittels eines Haftvermittlers mit einer Oberfläche des Kosmetikpads verbunden. Der Einsatz eines Haftvermittlers ist mit verfahrenstechnischen Schwierigkeiten, hohen Kosten und einer Beeinträchtigung des Absorptionsvermögens des Kosmetikpads verbunden. Nachteil beider Ausführungsformen ist überdies, dass die durchgehende Kunststoffbeschichtung die Zugänglichkeit des Faserverbundes verhindert. Die beschichtete Seite des Kosmetikpads steht somit nicht zur Aufnahme von Körper- oder Reinigungsflüssigkeiten zur Verfügung. Außerdem verursacht die durchgehende Kunststoffbeschichtung zum einen eine vom Verwender als unangenehm empfundene Versteifung des Pads und zum anderen den Nachteil sehr harter Produktränder.

EP-1283019-A1 offenbart ein zur Reinigung der Haut, insbesondere zum Peeling geeignetes Kosmetikpad umfassend eine Faserschicht, wobei zumindest eine Seite des Kosmetikpads oberflächenrauh ausgebildet ist. Die diskontinuierliche Beschichtung besteht aus in geschmolzenem Zustand auf zumindest eine Oberfläche des Kosmetikpads aufgebrachtem Kunststoff, insbesondere einem Hot-Melt. Von Nachteil sind die nur geringen abrasiven, da überwiegend runde Formen aufweisenden Oberflächen von in geschmolzenem Zustand aufgebrachten Kunststoffpartikeln. Überdies neigen die geschmolzenen Kunststoffpartikel dazu, in unerwünscht hohem Maße in den Faserverbund des Kosmetikpads einzudringen. Auch hat sich gezeigt, dass die Größe der Kunststoffpartikel nur unzureichend den Erfordernissen eines Peeling-Pads angepasst werden kann. Die in Beispielen 1 und 2 offenbarten Kunststoffpartikel wiesen bei einem Durchmesser von 1,28mm bzw. 1,36 mm eine Höhe von nur 0,08 mm bzw. 0,04 mm auf.

Es war eine Aufgabe der vorliegenden Erfindung, ein oberflächenraues zum Peeling geeignetes Kosmetikpad bereitzustellen, das die angeführten Nachteile überwindet.

Die Aufgabe wird erfindungsgemäß gelöst durch ein Kosmetikpad, das eine Faserschicht umfasst, wobei zumindest eine Seite des Kosmetikpads oberflächenrauh ausgebildet ist, indem dort ein partikuläres Schleifmittel aufgesintert ist.

Partikuläres Schleifmittel, das im Sinterverfahren aufgebracht wird, neigt kaum dazu, über das zur Fixierung notwendige Maß in die Faserstruktur einzudringen. Die Umkehrung der Abfolge der Zustandsformen des Schleifmittels von "erst geschmolzen dann fest", wie in EP-1283019-A1 beschrieben, nach "erst fest dann geschmolzen" gemäß vorliegender Erfindung ermöglicht außerdem, die Oberflächenrauigkeit des Schleifmittels zielgenauer einzustellen, insbesondere vergleichsweise rauer zu machen.

Der Sinterprozess erfordert nämlich im Gegensatz zum Stand der Technik kein vollständiges Er- bzw. Durchschmelzen des Kunststoffes; vielmehr werden die Kunststoffpartikel lediglich an der Oberfläche angeschmolzen.

Das Schleifmittel kann in vorteilhafter Weise aus einem thermoplastischen Schmelzklebepulver gebildet oder in ein Schmelzklebepulver eingearbeitet sein.

Das Schmelzklebepulver kann als pulverförmige Komponente, bspw. mittels eines Präzisionsstreuers über ein Vibrationssieb auf die eine Seite des Faservlieses, aus dem die späteren Wattepads herausgebildet werden, aufgebracht und dort thermisch fixiert werden. So kann das Pulver über einen vorzugsweise automatisch befüllten Trichter mit Hilfe eines Rakels in die Zwischenräume einer als Nadelwalze ausgebildeten Streuwalze zu bringen. Mit Hilfe eines Ausbürstsystems z.B. einem oszillierenden Nadelband oder einer Schwingbürste oder einer Ausbürstwalze, wird das Material aus den Zwischenräumen der Streuwalze ausgebürstet und auf dem darunter durchlaufenden Trägermaterial, das heißt dem Faservlies, aus dem die späteren Wattepads herausgebildet werden, gleichmäßig verteilt.

Vorteilhaft wäre auch der Einsatz der Schablonentechnik. Mittels dieser Technologie können verschiedene Designs und Muster auch mit verschiedenen Farben abgebildet werden.

Das Schmelzklebepulver wird vorzugsweise über einen Schneckenförderer einem rotierenden Sieb, der Schablone, zugeführt und vorzugsweise mittels eines Doppelrakelsystems durch die Schablone auf das darunter durchlaufende Trägermaterial gerakelt. Zum Erstellen von mehrfarbigen Mustern im Rapport werden mehrere Schablonen aneinandergereiht.

Die thermische Fixierung erfolgt vorteilhafterweise durch einen Sinterprozess in einem Ofen mittels konvektiver Wärmeübertragung. Denkbar ist auch die Verwendung von IR-Strahlung innerhalb einer IR-Sinterstrecke. Hierbei wird das Schleifmittel nicht aufgeschmolzen, sondern die Partikel werden unter weitgehender Beibehaltung der die oberflächenraue Struktur bildenden Partikelform jedoch unter Abrundung der Oberfläche der Partikel mit dem Wattepad und gegebenenfalls miteinander durch den Sinterprozess verbunden, indem nur die Oberfläche der Partikel auf- oder angeschmolzen wird.

Das vorteilhafterweise zu verwendende Schmelzklebepulver umfasst Polyethylen, vorzugsweise LDPE mit einem Schmelzbereich von 100-114 °C und/oder Polyamid, vorzugsweise Copolyamid mit einem Schmelzbereich von 110-127 °C, und/oder Polyester, vorzugsweise Copolyester mit einem Schmelzbereich von 105-115°C. Es weist eine Körnung von 1 - 500 μm, insbesondere von 1 - 100 μm, vorzugsweise von 1 - 65 μm auf. Es hat sich dabei als zweckmäßig und vorteilhaft erwiesen, wenn das Schleifmittel mit einem Flächengewicht von 5 - 50 g/m², insbesondere von 10 - 40 m² und vorzugsweise von 15 - 30 g/m² aufgebracht ist. Das Pulver kann farblos oder eingefärbt sein.

In einer besonders vorteilhaften Ausführungsform der Erfindung weisen die aufgesinterten Schleifmittelpartikel einen Durchmesser von 50-400 μm, insbesondere von 100-350 μm, ganz besonders bevorzugt von 150-300 μm auf. Hierunter wird die längste Erstreckung eines Schleifmittelpartikels verstanden projiziert auf die Ebene der flächenhaften Erstreckung des Kosmetikpads.

Bevorzugt weisen die aufgesinterten Schleifmittelpartikel eine Höhe von 50-400 μm, insbesondere von 100-350 μm, ganz besonders bevorzugt von 150-300 μm auf. Unter der Höhe wird die längste Erstreckung eines Schleifmittelpartikels senkrecht zur Ebene der flächenhaften Erstreckung der Faserschicht des Kosmetikpads verstanden.

In besonders vorteilhafter Ausführungsform der Erfindung beträgt das Verhältnis Durchmesser:Höhe der aufgesinterten Schleifmittelpartikel höchstens 10:1, insbesondere höchstens 7:1, ganz besonders höchstens 3:1 und vorzugsweise höchstens 1,5:1.

Es hat sich gezeigt, dass die vorstehend beschriebene Größe der aufgesinterten Schleifmittelpartikel besonders geeignet ist, einen effektiven als angenehm empfundenen Peeling-Effekt herbeizuführen. Die Sintertechnologie macht es möglich die Größe des Schleifmittels des Kosmetikpads im vorgenannten Bereich zu dimensionieren, da wie beschrieben im Sinterprozess lediglich die Oberfläche der Partikel leicht angeschmolzen wird.

Die Faserschicht des Kosmetikpads kann bis zu 100 Gew.-% aus Baumwollfasern bestehen.

In einer vorteilhaften Ausführungsform weist das Kosmetikpad Mikrostapelfasern, insbesondere einer Länge von mindestens 7mm auf.

Wenn vorstehend von Mikrostapelfasern die Rede ist, so werden hierunter synthetische Fasern einer Faserstärke von ≤ 1 dtex verstanden. Der Begriff der Mikrostapelfasern bringt dabei zum Ausdruck, dass für die Herstellung der Faservlieslage des Wattepads zuvor in einem separaten Herstellungsverfahren gebildete Mikrofasern einer bestimmten Länge oder eines bestimmten Längenbereichs verwendet werden. Es hat sich gezeigt, dass synthetische Mikrostapelfasern einem Wattepad zu größerer Weichheit und damit zu einer hervorragenden Anfühlungswahrnehmung beim Benutzer zu verhelfen vermögen. Überraschenderweise ist damit aber nicht ein Rückgang der Reinigungs- oder Abschminkwirkung verbunden, sondern im Gegenteil weisen erfindungsgemäße Wattepads mit oder bestehend aus synthetischen Mikrostapelfasern vorzugsweise einer Länge von wenigstens 7 mm, eine überlegene Reinigungs- und Abschminkwirkung auf.

Dies ist möglicherweise auf die aufgrund der Feinheit der Mikrostapelfasern zurückführbare große Oberfläche zurückzuführen, die zum Kontakt mit der zu reinigenden Hautoberfläche treten kann. Diese große Oberfläche begrenzt demzufolge auch eine riesige Anzahl von Mikrospalten und -öffnungen, welche zur Aufnahme von Unreinheiten, Hautschuppen oder Schminke dienen können.

Die Faserlänge der verwendbaren Mikrostapelfasern beträgt vorzugsweise 10 - 38 mm, insbesondere 15 - 32 mm.

Bei den Mikrostapelfasern kann es sich in weiterer Ausbildung der Erfindung um Polyester (PES) oder um Viskosefasern handeln. Die Oberfläche der Mikrostapelfasern sind vorzugsweise hydrophiliert. Auch kann die eine oder andere Oberfläche des Wattepads ein Prägemuster aufweisen. Solchenfalls kann das Prägemuster in an sich bekannter Weise, beispielsweise durch Kalanderprägung oder auch wie in EP-1106723-A2 oder WO-99/25318-A1 offenbart durch Wasserstrahlvernadelung erzeugt sein. Mit der Erzeugung des Prägemusters kann gleichzeitig eine Verfestigung des Vlieses einhergehen. Auch eine Vliesverfestigung durch den Zusatz wärmeschmelzbarer Bindefasern oder durch den Zusatz von chemischen Bindemitteln, wie z. B. wässrige Polymerdispersionen, z. B. Polyacrylate, Polyvinylacetate, Polyvinylalkohole, Latices oder Bindemittel auf Basis von Lösungsmitteln oder Polyurethane oder Streukleber/Schmelzklebepulver aus z. B. Polyamid, Polyethylen, Ethylenvinylacetat, Polyurethan oder Polyester, sind denkbar.

Das Flächengewicht des erfindungsgemäßen Kosmetikpads liegt vorzugsweise bei 40 - 300 g/m², insbesondere bei 60 - 250 g/m² und vorzugsweise bei 120 - 250 g/m² und besonders bevorzugtermaßen bei 150 - 250 g/m².

Insbesondere wenn das mikrofaserhaltige kosmetische Wattepad zur Flüssigkeitsaufnahme ausgebildet werden soll, wenn es sich also bspw. gut zur Aufnahme einer gesichtsreinigenden Lösung eignen soll oder zum Abschminken unter Verwendung eines einen hohen Flüssigkeitsanteil aufweisenden Abschminkmittels, erweist es sich als vorteilhaft, wenn das Wattepad zusätzlich bis 72 Gew.-% Baumwollfasern, insbesondere 15 - 65 Gew.-% und weiter insbesondere 50 - 65 Gew.-% Baumwollfasern enthält. Hierbei handelt es sich vorteilhafterweise um Baumwollkämmlinge. Das Wattepad kann solche Baumwollfasern umfassen, die zu wenigstens 0,2 Gew.-% mit einem Weichmacher beaufschlagt sind. Dieser Weichmacher kann ein Fettsäurekondensationsprodukt und/oder funktionelle Polydimethylsiloxane und/oder Polyethylene umfassen.

Des Weiteren erweist es sich als besonders vorteilhaft im Hinblick auf die Erreichung einer hohen inneren Festigkeit bei dem erfindungsgemäßen Kosmetikpad, wenn zusätzlich wärmeschmelzbare Bindefasern, vorzugsweise zu 10 - 20 Gew.-%, enthalten sind, und mit diesen Bindefasern eine thermische Verfestigung des Wattepads durchgeführt wurde. Der Anteil wärmeschmelzbarer Bindefasern beträgt insbesondere 12 - 18 Gew.-% und weiter insbesondere 12 - 15 Gew.-% bezogen auf die Masse des Wattepads.

In weiterer Ausbildung der Erfindung kann es sich bei den Bindefasern um Mehrkomponentenfasern, insbesondere Bikomponenten-Fasern handeln mit einer höher schmelzenden Trägerkomponente und einer niedriger schmelzenden Komponente.

Die Mehrkomponentenfasern, insbesondere Bikomponentenfasern, haben in vorteilhafter Weise eine Faserstärke von 1,3 - 10 dtex, insbesondere von 1,3 - 3 dtex und eine Faserlänge von 3 bis 60 mm. Vorteilhafterweise kommen Kern/Mantel-Fasern oder Seite-an-Seite-Fasern zum Einsatz.

Es hat sich als vorteilhaft erwiesen, wenn Bikomponentenfasern mit einem Copolyester (CO-PES) als niedrig schmelzender Komponente und Polyester (PES) als höher schmelzender Komponente verwendet werden.

In weiterer Ausbildung der Erfindung von besonderer Bedeutung ist der Schmelzpunkt der wärmeschmelzbaren Bindefasern oder der niedrig schmelzenden Komponente (z.B. CO-PES) der Mehrkomponentenfasern geringer als der Schmelzpunkt der Mikrostapelfasern. Bspw. könnten Mikrostapelfasern aus einem Polyestermaterial verwendet werden mit einem Schmelzpunkt von etwa 256°C und CO-PES/PES-Bikomponentenfasern mit einem Schmelzpunkt der niedrig schmelzenden Komponente CO-PES von 110°C und der höher schmelzenden Komponente PES von 255°C. Solchenfalls könnte eine thermische Verfestigung des Faservlieses durchgeführt werden, ohne die höher schmelzende Komponente der Bikomponentenfasern und die Mikrostapelfasern thermisch zu verändern.

Das erfindungsgemäße Wattepad ist vorzugsweise derart verdichtet, dass es eine Längsfestigkeit bzw. Höchstzugkraft in Längsrichtung (Maschinenrichtung) aufweist von 5 - 30 N/25mm, insbesondere 10 - 25 N/25mm und vorzugsweise > 15 N/25mm und eine Höchstzugkraft in Querrichtung (quer zur Maschinenrichtung) von 5 - 30 N/25mm, insbesondere 8 - 20 N/25mm und vorzugsweise > 10 N/25mm auf. Diese Höchstzugkraft kann unter Verwendung einer genormten Zugprüfmaschine nach DIN 51221 nach der folgenden Prüfmethode ermittelt werden: Es werden aus dem zu prüfenden Wattepad, und zwar aus einem mittleren Bereich, Proben einer Einspannbreite von 25 mm und einer Einspannlänge von 30 mm genommen. Die in Klemmaufnahmen der normierten Zugprüfmaschine lotrecht eingespannten Proben werden dann mit einer Prüfgeschwindigkeit von 100 mm/min in der Ebene Ihrer Erstreckung auseinanderbewegt und dabei wird die in dieser Richtung wirkende Zugkraft gemessen. Unter der Höchstzugkraft wird diejenige maximale Kraft verstanden, bei der das Wattepad zerreist. Wenn zuvor höhere Kraftspitzen im Zuge der Dehnung gemessen werden, so stellen diese die Höchstzugkraft im Sinne dieser Prüfung dar. Man kann vorteilhafterweise bei Messungen der Längs- und der Querrichtung, welche der Maschinenrichtung bzw. der Richtung quer hierzu entspricht, verschiedene, insbesondere fünf Einzelmessungen vornehmen und deren Mittelwert berechnen.

Das erfindungsgemäße Wattepad weist ferner eine Dicke von vorzugsweise 0,5 - 4,5 mm auf, die unter einem spezifischen Messdruck von 0,5 kPa auf einer Tasterfläche von 25 cm$^2$ einer Probe des Prüflings ermittelt wurde. Das Prüfverfahren entspricht DIN EN ISO 9073-2 (Prüfverfahren für Vliesstoffe, Bestimmung der Dicke).

Des Weiteren kann das Absorptionsvermögen erfindungsgemäßer Wattepads bestimmt werden, und zwar wird hierfür entsprechend PH.EUR.1997, Monografie Verbandwatte aus Baumwolle, ein Test der Saugfähigkeit unter Bestimmung der Absinkdauer eines mit zu testenden Prüflingen befüllten Drahtkörbchens in einer Flüssigkeit ermittelt. Das hierzu verwendbare Drahtkörbchen ist ein zylindrischer Korb aus Kupferdraht mit einem Drahtdurchmesser von 0,4 mm. Die Höhe beträgt 80 mm, der Durchmesser 50 mm, die Maschenweite 15 - 20 mm und die Masse 2,7 +/- 0,3 g. Ferner findet ein Becherglas mit Durchmesser 11 - 12 cm Verwendung.

Zu testende Wattepads werden, bis eine Prüfmenge von 5 g vorhanden ist, in das Drahtkörbchen eingelegt. Zuvor wurde der Korb auf 0,01 g genau gewogen (M1). Die 5 g bilden die Masse M2. Das Becherglas wird mit demineralisiertem Wasser bis zu einer Höhe von etwa 100 mm gefüllt und der gefüllte Korb wird aus einer Höhe von 10 mm auf das Wasser fallen gelassen. Mit einer Stopuhr wird die Zeit bis zum Absinken unter die Wasseroberfläche bestimmt. Unmittelbar nach der Bestimmung der Absinkdauer wird der Korb aus dem Wasser gehoben und zum Abtropfen 30 s lang in seiner Längsachse horizontal gehalten. Nach Ablauf der Abtropfzeit wird der Korb in ein tariertes Becherglas (M3) gegeben und auf 0,01 g genau gewogen (M4).

Das Wasserhaltevermögen ergibt sich

$$\text{in g/g} = \frac{M4 - (M2 + M3)}{M2 - M1}$$

Die Absinkdauer und das Wasserhaltevermögen werden als Mittelwert aus drei Bestimmungen angegeben. Für bevorzugte Wattepads beträgt die Absinkdauer maximal 15 sec und das Wasserhaltevermögen beträgt wenigstens 10 g/g. Dies lässt sich durch den Anteil absorbierender Fasern und/oder durch Zusatz von Hydrophilierungsmittel erreichen.

Es versteht sich, dass die erfindungsgemäßen Kosmetikpads hinsichtlich Größe und Form beliebige Formate aufweisen können. Denkbar sind insbesondere kreisrunde, ovale, quadratische und rechteckige Formen.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:

Figur 1: eine Darstellung eines erfindungsgemäßen Kosmetikpads

Figur 2: eine Schnittansicht des Kosmetikpads aus Figur 1

Figur 3: eine vergrößerte Teilansicht der Schnittansicht aus Figur 2

Figur 4: eine weitere Ausführungsform eines erfindungsgemäßen Kosmetikpads mit einem als Streifenmuster aufgetragenen Schleifmittel

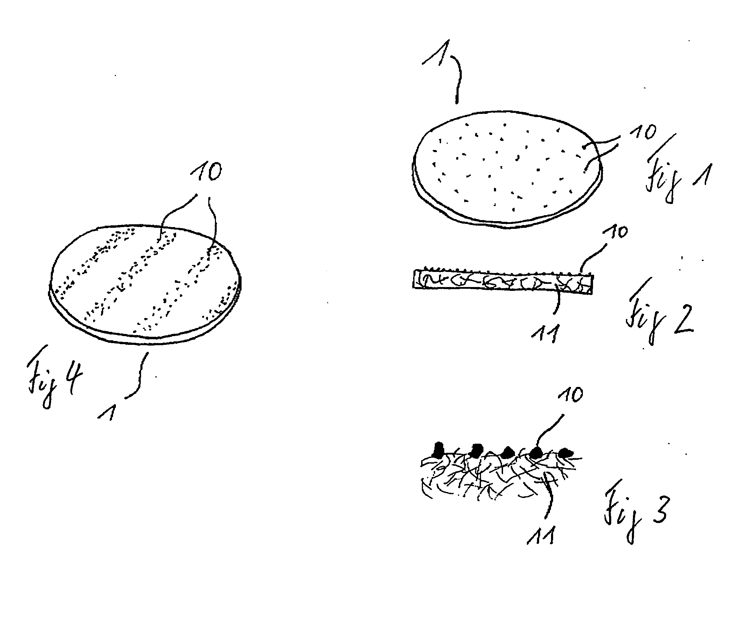

Figur 1 zeig eine perspektivische Ansicht des erfindungsgemäßen Kosmetikpads 1. Figur 2 ist die Darstellung einer Schnittansicht. Das Pad besteht aus einem Wattepad aus Baumwollfasern 11, auf dessen eine Seite ein pulverförmiges thermoplastisches Schleifmittel aufgestreut und im Sinterverfahren auf dem Wattepad fixiert wurde.

Die Körnung des verwendeten Schleifmittelpulvers beträgt 50-300 $\mu$m, das in einer Menge von 10-40 g/m$^2$ aufgetragen ist. Das Schleifmittelpulver kann farblos oder eingefärbt sein.

Vorzugsweise besteht das Schleifmittel aus einem Copolyamid mit einem Schmelzbereich von 110-127°C.

Die schematische Darstellung in Figur 3 zeigt, dass die Schleifmittelpartikel 10 zwar auf der Faseroberfläche fixiert sind, jedoch nicht zu tief in den Faserverbund eindringen. Auf diese Weise können die Schleifmittelpartikel den Peeling-Effekt bei der bestimmungsgemäßen Anwendung des Pads uneingeschränkt bewirken.

Die äußere Oberfläche der Partikel selbst ist auch nach dem Sinterprozess mit Ecken und Kanten versehen, die durch das Anschmelzen während der Sinterung nur eine leichte Abrundung erfahren haben, so dass keine Gefahr der zu starken Beanspruchung und Reizung der Haut gegeben ist.

Zwischen den Schleifmittelpartikeln ist die Faseroberfläche frei zugänglich, so dass auch die mit Schleifmittel versehene Oberfläche zur Absorption von Reinigungs- oder Körperflüssigkeit zur Verfügung steht.

Figur 4 zeigt eine weitere Ausführungsform der Erfindung. Das Schleifmittelpulver 10 ist auf der einen Seite des Wattepads in einem Streifenmuster aufgetragen.

**Patentansprüche**

1.  Zur Reinigung der Haut insbesondere zum Peeling geeignetes Kosmetikpad umfassend eine Faserschicht, wobei zumindest eine Seite des Kosmetikpads oberflächenrauh ausgebildet ist, indem dort ein partikuläres Schleifmittel aufgesintert ist.

2.  Kosmetikpad nach Anspruch 1, **dadurch gekennzeichnet, dass** das Schleifmittel ein thermoplastisches Schmelzklebepulver aus Polyamid und/oder ein Polyethylen und/oder einen Polyester umfasst.

3.  Kosmetikpad nach Anspruch 1 oder 2, **dadurch gekennzeichnet, dass** die Partikel des aufgesinterten Schleifmittels einen Durchmesser von 50 - 400 $\mu$m, insbesondere von 100 - 350 $\mu$m, insbesondere von 150 - 300 $\mu$m aufweisen.

4.  Kosmetikpad nach Anspruch 1, 2 oder 3, **dadurch gekennzeichnet, dass** die Partikel des aufgesinterten Schleifmittels eine Höhe senkrecht zur Ebene der flächenhaften Erstreckung der Faserschicht des Kosmetikpads von 50 - 400 $\mu$m, insbesondere von 100 - 350 $\mu$m, insbesondere von 150 - 300 $\mu$m aufweisen.

5.  Kosmetikpad nach einem oder mehreren der vorgenannten Ansprüche, **dadurch gekennzeichnet, dass** das Schleifmittel mit einem Flächengewicht von 5-50 g/m$^2$, insbesondere von 10-40 g/m$^2$ und vorzugsweise von 15-30 g/m$^2$ aufgebracht ist.

6.  Kosmetikpad nach einem oder mehreren der vorgenannten Ansprüche, **dadurch gekennzeichnet, dass** die Faserschicht einen Vliesstoff umfasst.

7.  Kosmetikpad nach einem oder mehreren der vorgenannten Ansprüche, **dadurch gekennzeichnet, dass** die Faserschicht synthetische Mikrostapelfasern insbesondere einer Länge von mindestens 7mm umfasst oder daraus besteht.

8.  Kosmetikpad nach Anspruch 7, **dadurch gekennzeichnet, dass** es sich bei den Mikrostapelfasern um Polyester (PES)- oder um Viskosefasern handelt.

9.  Kosmetikpad nach einem oder mehreren der vorgenannten Ansprüche, **dadurch gekennzeichnet, dass** das Flächengewicht des Kosmetikpads 40 - 300 g/m$^2$, insbesondere 60 - 250 g/m$^2$, insbesondere

120 - 250 g/m² und vorzugsweise 150 - 250 g/m² beträgt.

10. Kosmetikpad nach einem oder mehreren der vorgenannten Ansprüche, **dadurch gekennzeichnet, dass** der Anteil der Mikrostapelfasern 15 - 85 Gew.-%, insbesondere 15 - 65 Gew.-% und insbesondere 20 - 30 Gew.-% beträgt.

11. Kosmetikpad nach einem oder mehreren der vorgenannten Ansprüche, **dadurch gekennzeichnet, dass** zusätzlich bis 72 Gew.-%, insbesondere 15 - 65 Gew.-% und weiter insbesondere 50 - 65 Gew.-% Baumwollfasern enthalten sind.

12. Kosmetikpad nach einem oder mehreren der vorgenannten Ansprüche, **dadurch gekennzeichnet, dass** die Baumwollfasern Baumwollkämmlinge sind.

13. Kosmetikpad nach einem oder mehreren der vorgenannten Ansprüche, **dadurch gekennzeichnet, dass** zusätzlich wärmeschmelzbare Bindefasern enthalten sind, insbesondere in einem gewichtsprozentualen Anteil von 10 - 20 Gew.-%, insbesondere 12 - 18 Gew.-% und weiter insbesondere 12 - 15 Gew.-%.

14. Kosmetikpad nach einem oder mehreren der vorgenannten Ansprüche, **dadurch gekennzeichnet, dass** die Bindefasern Mehrkomponentenfasern, insbesondere Bikomponenten-Fasern sind.

15. Kosmetikpad nach einem oder mehreren der vorgenannten Ansprüche, **dadurch gekennzeichnet, dass** die Mehrkomponenten-Fasern eine Faserstärke von 1,3 - 10 dtex, insbesondere von 1,3 - 3,0 dtex und eine Faserlänge von 3 - 60 mm haben.

16. Kosmetikpad nach einem oder mehreren der vorgenannten Ansprüche, **dadurch gekennzeichnet, dass** es sich bei den Bikomponentenfasern um Copolyester (CO-PES) / Polyester (PES)- Bikomponentenfasern handelt.

17. Kosmetikpad nach einem oder mehreren der vorgenannten Ansprüche, **dadurch gekennzeichnet, dass** der Schmelzpunkt der wärmeschmelzbaren Bindefasern oder der niedrig schmelzenden Komponente der Mehrkomponentenfasern geringer ist als der Schmelzpunkt der Mikrostapelfasern.

18. Verwendung eines Kosmetikpads nach einem oder mehreren der vorstehenden Ansprüche zum Reinigen und/oder Abschminken der Haut.

19. Verfahren zur Herstellung eines oberflächenrau ausgebildeten Kosmetikpads nach einem der Anprüche 1-17 umfassend die Verfahrensschritte

    - Bereitstellen einer Faserschicht
    - Aufbringen von partikulärem Schmelzklebepulver auf eine Seite der Faserschicht
    - Thermisches Verbinden der Partikel mit der Faserschicht durch einen Sinterprozess.

20. Verfahren nach Anspruch 19 **dadurch gekennzeichnet, dass** das partikuläre Schmelzklebepulver auf eine Seite der Faserschicht im wesentlichen gleichförmig aufgestreut wird.

21. Verfahren nach Anspruch 19 **dadurch gekennzeichnet, dass** das partikuläre Schmelzklebepulver auf eine Seite der Faserschicht mittels Schablonentechnik ungleichförmig aufgebracht wird.

22. Verfahren nach Anspruch 19, 20 oder 21, **dadurch gekennzeichnet, dass** das thermische Verbinden mittels Heißluft oder Infrarot Strahlung, erfolgt.

## Claims

1. A cosmetic pad suitable for cleaning the skin, in particular for peeling, including a fiber layer, wherein at least one side of the cosmetic pad has a rough surface which is formed by sintering a particulate abrasive material thereto.

2. Cosmetic pad of claim 1 **characterized in that** the abrasive material includes a meltable thermoplastic glue powder containing polyamide and/or polyethylene and/or a polyester.

3. Cosmetic pad according to claim 1 or 2 **characterized in that** the particles of the sintered abrasive material have a diameter of 50-400 $\mu$m, in particular 100-350 $\mu$m, in particular 150 - 300 $\mu$m.

4. Cosmetic pad according to claim 1, 2 or 3 **characterized in that** the particles of the sintered abrasive material have a height perpendicular to the plane of flat extension of the fiber layer of the cosmetic pad of 50-400 $\mu$m, in particular 100-350 $\mu$m, in particular 150-300$\mu$m.

5. Cosmetic pad according to any one or more of the previous claims, **characterized in that** the abrasive material is applied with a basis weight of 5-50 g/m², in particular 10 - 40 g/m² and preferably 15 - 30 g/m².

6. Cosmetic pad according to any one or more of the preceding claims **characterized in that** the fiber layer includes a non-woven material.

7. Cosmetic pad according to any one or more of the preceding claims **characterized in that** the fiber layer consists essentially of or includes synthetic micro staple fibers, in particular having a length of at least 7 mm.

8. Cosmetic pad according to claim 7 **characterized in that** the micro staple fibers are polyester (PES) or viscose fibers.

9. Cosmetic pad according to any one or more of the preceding claims **characterized in that** the basis weight of the cosmetic pad is 40-300 g/m$^2$, in particular 60-250 g/m$^2$, in particular 120-250 g/m$^2$ and preferably 150-250 g/m$^2$.

10. Cosmetic pad according to any one or more of the preceding claims **characterized in that** the fraction of micro staple fibers is 15-85 % per weight, in particular 15-65 % per weight and preferably 20-30 % per weight.

11. Cosmetic pad according to any one or more of the preceding claims **characterized in that** in addition cotton fibers are included up to 72 % per weight, in particular 15-65 % per weight and further in particular 50-65 % per weight.

12. Cosmetic pad according to any one or more of the preceding claims **characterized in that** the cotton fibers are cotton noils.

13. Cosmetic pad according to any one or more of the preceding claims **characterized in that** in addition heat meltable binding fibers are included, in particular with a weight percent fraction of 10-20 % per weight, in particular 12-18 % per weight and further in particular 12-15 % per weight.

14. Cosmetic pad according to any one or more of the preceding claims **characterized in that** the binding fibers are multi-component fibers, in particular bi-component fibers.

15. Cosmetic pad according to any one or more of the preceding claims **characterized in that** the multi-component fibers have a fiber thickness of 1,3 to 10 dtex, in particular 1,3 to 3,0 dtex and a fiber length of 3-60 mm.

16. Cosmetic pad according to any one or more of the preceding claims **characterized in that** the bicomponent fibers are co-polyester (CO-PES)/ polyester (PES) bicomponent fibers.

17. Cosmetic pad according to any one or more of the preceding claims **characterized in that** the melting temperature of the heat meltable binding fibers or of the low melting temperature component of the multi-component fibers is less than the melting point of the micro staple fibers.

18. Use of a cosmetic pad according to any one or more of the preceding claims for the cleaning and/or make-up removal of skin.

19. Method for the manufacture of a cosmetic pad having a roughened surface according to any one or more of the claims 1 to 17, the method including the following steps:

   - Preparation of a fiber layer
   - Applying of meltable gluing powder particles onto one side of the fiber layer
   - Thermal bonding of the particles to the fiber layer by a sintering process.

20. Method according to claim 19, **characterized in that** the meltable glue powder particles are distributed on one side of the fiber layer in a substantially uniform fashion.

21. Method according to claim 19, **characterized in that** the meltable glue powder particles are introduced onto one side of the fiber layer in a non-uniform fashion using a template.

22. Method according to claim 19, 20 or 21, **characterized in that** the thermal bonding is effected by means of hot air or infrared radiation.

## Revendications

1. Rondelle cosmétique apte à nettoyer la peau, en particulier à la gommer, comprenant une couche fibreuse, au moins un côté de la rondelle cosmétique présentant une surface rugueuse, de sorte qu'un produit abrasif particulaire y est aggloméré par frittage.

2. Rondelle cosmétique selon la revendication 1, **caractérisée en ce que** le produit abrasif comprend une poudre fusible thermoplastique de polyamide et/ou un polyéthylène et/ou un polyester.

3. Rondelle cosmétique selon la revendication 1 ou 2, **caractérisée en ce que** les particules du produit abrasif aggloméré par frittage présentent un diamètre de 50 à 400 $\mu$m, en particulier de 100 à 350 $\mu$m, en particulier de 150 à 300 $\mu$m.

4. Rondelle cosmétique selon la revendication 1, 2 ou 3, **caractérisée en ce que** les particules du produit abrasif aggloméré par frittage présentent une hauteur, perpendiculairement au plan de l'étendue plane de la couche fibreuse de la rondelle cosmétique, allant de 50 à 400 $\mu$m, en particulier de 100 à 350 $\mu$m,

en particulier de 150 à 300 μm.

**5.** Rondelle cosmétique selon une ou plusieurs des revendications précédentes, **caractérisée en ce que** le produit abrasif est appliqué avec un poids par unité de surface de 5 à 50 g/m², en particulier de 10 à 40 g/m² et de préférence de 15 à 30 g/m².

**6.** Rondelle cosmétique selon une ou plusieurs des revendications précédentes, **caractérisée en ce que** la couche fibreuse comprend un non-tissé.

**7.** Rondelle cosmétique selon une ou plusieurs des revendications précédentes, **caractérisée en ce que** la couche fibreuse comprend ou est composée par des microfibres discontinues synthétiques, en particulier d'une longueur d'au moins 7 mm.

**8.** Rondelle cosmétique selon la revendication 7, **caractérisée en ce que**, pour ce qui est des microfibres discontinues, il s'agit de fibres de polyester (PES) ou de viscose.

**9.** Rondelle cosmétique selon une ou plusieurs des revendications précédentes, **caractérisée en ce que** le poids par unité de surface de la rondelle cosmétique va de 40 à 300 g/m², en particulier de 60 à 250 g/m², en particulier de 120 à 250 g/m² et de préférence de 150 à 250 g/m².

**10.** Rondelle cosmétique selon une ou plusieurs des revendications précédentes, **caractérisée en ce que** la part des microfibres discontinues va de 15 à 85 % en poids, en particulier de 15 à 65 % en poids et en particulier de 20 à 30 % en poids.

**11.** Rondelle cosmétique selon une ou plusieurs des revendications précédentes, **caractérisée en ce qu'**elle comprend en plus jusqu'à 72 % en poids, en particulier de 15 à 65 % en poids, et encore plus particulièrement de 50 à 65 % en poids de fibres de coton.

**12.** Rondelle cosmétique selon une ou plusieurs des revendications précédentes, **caractérisée en ce que** les fibres de coton sont des débourrures de coton.

**13.** Rondelle cosmétique selon une ou plusieurs des revendications précédentes, **caractérisée en ce qu'**elle comprend en plus des fibres de liaison thermofusibles, en particulier dans une part en pourcentage en poids comprise entre 10 et 20 % en poids, en particulier entre 12 et 18 % en poids, et encore plus particulièrement entre 12 et 15 % en poids.

**14.** Rondelle cosmétique selon une ou plusieurs des revendications précédentes, **caractérisée en ce que** les fibres de liaison sont des fibres à plusieurs constituants, en particulier des fibres à deux constituants.

**15.** Rondelle cosmétique selon une ou plusieurs des revendications précédentes, **caractérisée en ce que** les fibres à plusieurs constituants ont une épaisseur de fibre comprise entre 1,3 et 10 dtex, en particulier entre 1,3 et 3,0 dtex et une longueur de fibre comprise entre 3 et 60 mm.

**16.** Rondelle cosmétique selon une ou plusieurs des revendications précédentes, **caractérisée en ce que**, pour ce qui est des fibres à deux constituants, il s'agit de fibres à deux constituants copolyester (CO-PES) /polyester (PES).

**17.** Rondelle cosmétique selon une ou plusieurs des revendications précédentes, **caractérisée en ce que** le point de fusion des fibres de liaison thermofusibles ou des constituants à bas point de fusion des fibres à plusieurs constituants est plus bas que le point de fusion des microfibres discontinues.

**18.** Utilisation d'une rondelle cosmétique selon une ou plusieurs des revendications précédentes pour nettoyer et/ou démaquiller la peau.

**19.** Procédé de fabrication d'une rondelle cosmétique réalisée rugueuse en surface selon l'une des revendications 1 à 17, comprenant les étapes suivantes :

    - préparation d'une couche fibreuse
    - application d'une poudre fusible particulaire sur un côté de la couche fibreuse
    - liaison thermique des particules à la couche fibreuse par un procédé de frittage.

**20.** Procédé selon la revendication 19, **caractérisé en ce que** la poudre fusible particulaire est répartie de façon essentiellement uniforme sur un côté de la couche fibreuse.

**21.** Procédé selon la revendication 19, **caractérisé en ce que** la poudre fusible particulaire est appliquée de façon non uniforme sur un côté de la couche fibreuse par la technique du pochoir.

**22.** Procédé selon la revendication 19, 20 ou 21, **caractérisé en ce que** la liaison thermique s'effectue par de l'air chaud ou par rayonnement infrarouge.

Fig. 1

Fig. 2

Fig. 3

Fig. 4

**IN DER BESCHREIBUNG AUFGEFÜHRTE DOKUMENTE**

**In der Beschreibung aufgeführte Patentdokumente**

- EP 1136024 A2
- EP 1283019 A1
- EP 1106723 A2
- WO 9925318 A1